# FASCICULE DE BREVET EUROPEEN

(11) **EP 3 041 441 B1**
(45) Date de publication et mention de la délivrance du brevet: **11.10.2017**
(21) Numéro de dépôt: 14759021.0
(22) Date de dépôt: 30.07.2014
(51) Int. Cl.: A61F 5/01

(54) **ORTHESE DE MAIN POUR LE MAINTIEN DU POUCE NOTAMMENT EN CAS DE RHIZARTHROSE**
HANDORTHESE ZUR UNTERSTÜTZUNG DES DAUMENS, INSBESONDERE BEI RHIZARTHROSE
HAND ORTHOSIS FOR SUPPORTING THE THUMB, IN PARTICULAR IN CASE OF RHIZARTHROSIS

(30) Priorité: 02.09.2013 FR 1358389
(43) Date de publication de la demande: 13.07.2016
(73) Titulaire: Millet Innovation, 26270 Loriol sur Drome (FR)
(72) Inventeur: GRANGE, Odile, 26400 Allex (FR)
(74) Mandataire: de Roquemaurel, Bruno
(86) Numéro de dépôt international: PCT/FR2014/051980
(87) Numéro de publication internationale: WO 2015/028734

(56) Documents cités:
- WO-A1-2013/001083
- DE-A1- 4 103 383
- FR-A1- 2 822 371
- FR-A1- 2 892 298
- US-A1- 2003 191 421
- US-B1- 6 702 772
- "Material Data Book", , 1 janvier 2003 (2003-01-01), XP055159291, Extrait de l'Internet: URL:www.-mdp.eng.cam.ac.uk [extrait le 2014-12-17]

## Description

La présente invention concerne une orthèse de pouce. La présente invention vise notamment à soulager les personnes souffrant de rhizarthrose.

Avec l'âge, un grand nombre de personnes souffrent d'arthrose, en particulier de l'articulation entre le pouce et le poignet. Cette affection appelée "rhizarthrose", touche principalement les femmes, et se manifeste par poussées très douloureuses. La nuit, des mouvements aléatoires du pouce peuvent entrainer des douleurs au point de priver de sommeil. Cette affection peut concerner l'articulation entre le trapèze et le métacarpe du pouce et/ou l'articulation entre le scaphoïde et le trapèze et le trapézoïde. Cette affection peut également se traduire par une instabilité ou une subluxation de l'articulation entre le trapèze et le métacarpe du pouce.

La rhizarthrose est généralement traitée par des médicaments et/ou par des accessoires d'orthopédie. Certains de ces accessoires se présentent sous la forme d'orthèses rigides visant à immobiliser totalement l'articulation en cause. Ces orthèses sont donc portées principalement la nuit. En immobilisant l'articulation, elles suppriment la douleur lorsque l'articulation est en crise. Elles évitent également une certaine fatigue de l'articulation en empêchant des mouvements involontaires.

Le principal inconvénient de ces orthèses réside dans leur rigidité qui empêche un usage normal du pouce et en particulier de la fonction essentielle de la main qu'est la fonction de pince. Ces orthèses présentent également l'inconvénient de devoir être réalisées sur mesure, par exemple par thermoformage sur la main.

Il existe également des orthèses élastiques visant également à immobiliser l'articulation. Or il s'avère que la fonction de pince de la main produit une importante démultiplication des efforts dans la chaine articulaire entre le pouce et le poignet. Ainsi, la force exercée par la pince est multipliée par un facteur de douze sur l'articulation carpo-métacarpienne. Il en résulte que ces orthèses doivent appliquer une forte pression de contention à la base du poignet, notamment pour contenir les mouvements liés à une éventuelle subluxation de l'articulation. Ces orthèses doivent donc être également réalisées sur mesure.

Par ailleurs, il existe des orthèses se présentant sous la forme d'un gantelet, également réalisé sur mesure par thermoformage sur la main.

Toutes les orthèses précédemment décrites présentent les inconvénients non seulement de devoir être réalisées sur mesure, mais également de couvrir le pli entre le pouce et la paume. Il en résulte que seule l'articulation interphalangienne du pouce n'est pas bloquée.

Les documents US6702772 et US2003/0191421 décrivent des orthèses de main pour le maintien du pouce telles que décrites ci-dessus.

Le document FR2822371 décrit une orthèse de main comprenant une pièce principale réalisée en un matériau élastique et comportant une première partie en forme de manchon ajustée à une partie du pouce s'étendant jusqu'à la zone de pli entre le pouce et la paume de la main, une seconde partie en forme de manchon ajustée au poignet, et une troisième partie reliant les première et seconde parties, conformée pour envelopper une zone de la main s'étendant le long du métacarpien du pouce entre le dos et la paume de la main, et comportant une ouverture pour le passage de la paume de la main.

Il est souhaitable de réaliser une orthèse de pouce apte à soulager l'articulation carpo-métacarpienne, mais sans entraver la fonction de pince de la main. Il est également souhaitable de réaliser une orthèse de pouce qui ne nécessite pas d'être réalisée sur mesure, mais seulement en quelques tailles standard, sans nécessiter d'ajustement final.

Des modes de réalisation concernent une orthèse de main pour le maintien du pouce, l'orthèse comprenant une pièce principale réalisée en un matériau élastique et comportant : une première partie en forme de manchon ajustée à une partie du pouce s'étendant jusqu'à la zone de pli entre le pouce et la paume de la main, une seconde partie en forme de manchon ajustée au poignet, et une troisième partie reliant les première et seconde parties, conformée pour envelopper une zone de la main s'étendant le long du métacarpien du pouce entre le dos et la paume de la main, et comportant une ouverture pour le passage de la paume de la main. Selon l'invention, la seconde partie est assemblée par une ligne de couture pour prendre la forme d'un manchon, et la pièce principale est conformée de manière à ne pas couvrir pas la zone de pli entre le pouce et la paume de la main et est réalisée en un matériau présentant dans une direction correspondant à un axe longitudinal de la première partie un module de Young compris entre 0,5 Mpa et 1 Mpa.

Selon un mode de réalisation, la première partie est conformée pour couvrir l'articulation entre les deux phalanges du pouce.

Selon un mode de réalisation, la pièce principale comprend deux couches de tissu élastique assemblées par collage.

Selon un mode de réalisation, chacune des deux couches est réalisée dans un tissu comprenant entre 75 et 85% en poids de polyamide et entre 15 et 25% en poids d'élasthanne.

Selon un mode de réalisation, chacune des deux couches présente un grammage de 155 g/m2, et/ou une épaisseur comprise entre 0,5 et 0,7 mm.

Selon un mode de réalisation, chacune des deux couches présente une élasticité de 85% à 115% dans le sens de la chaine et de 65% à 95% dans le sens de la trame.

Selon un mode de réalisation, l'orthèse comprend une plaquette en un matériau apte à assurer une fonction d'amortissement, la plaquette étant fixée à l'intérieur des seconde et troisième parties en une zone couvrant l'articulation entre le métacarpien du pouce et le poignet.

Selon un mode de réalisation, la plaquette est réalisée en un gel polymère réticulé.

Selon un mode de réalisation, la plaquette présente une épaisseur comprise entre 0,4 et 1 mm.

Selon un mode de réalisation, la plaquette est collée sur une pièce de recouvrement en tissu élastique qui est assemblée avec la pièce principale par une couture, de sorte que la plaquette soit prise en sandwich entre la pièce principale et la pièce de recouvrement.

Selon un mode de réalisation, la plaquette est cousue avec la pièce de recouvrement sur la pièce principale.

Selon un mode de réalisation, la pièce principale est ajustée à la main et au pouce pour maintenir les articulations du pouce dans une configuration de la main où tous les muscles de la main sont relâchés et en l'absence de forces extérieures s'exerçant sur les articulations du pouce.

Selon un mode de réalisation, la pièce principale est réalisée en un matériau présentant une épaisseur comprise entre 1 mm et 1,4 mm.

Des exemples de réalisation de l'invention seront décrits dans ce qui suit, à titre non limitatif en relation avec les figures jointes parmi lesquelles :
les figures 1A, 1B, 1C représentent schématiquement les articulations carpo-métacarpiennes du pouce, subissant différentes affections susceptibles d'être soulagées par l'orthèse selon l'invention,
la figure 2 représente la face dorsale d'une main munie d'une orthèse selon un mode de réalisation,
la figure 3 représente une partie de la face externe de l'orthèse,
la figure 4 représente une partie de la face interne de l'orthèse en configuration retournée,
la figure 5 représente la face interne d'une main dans une configuration posée sur son tranchant, muscles relâchés,
la figure 6 représente la face interne de la main dans la configuration présentée sur la figure 5, munie de l'orthèse des figures 2 à 4,
la figure 7 est une vue en coupe d'une partie de l'orthèse, selon un mode de réalisation,
la figure 8 représente la forme d'une pièce à plat à partir de laquelle l'orthèse peut être réalisée, selon un mode de réalisation,
les figures 9A et 9B sont des courbes en fonction du temps illustrant les performances de l'orthèse.

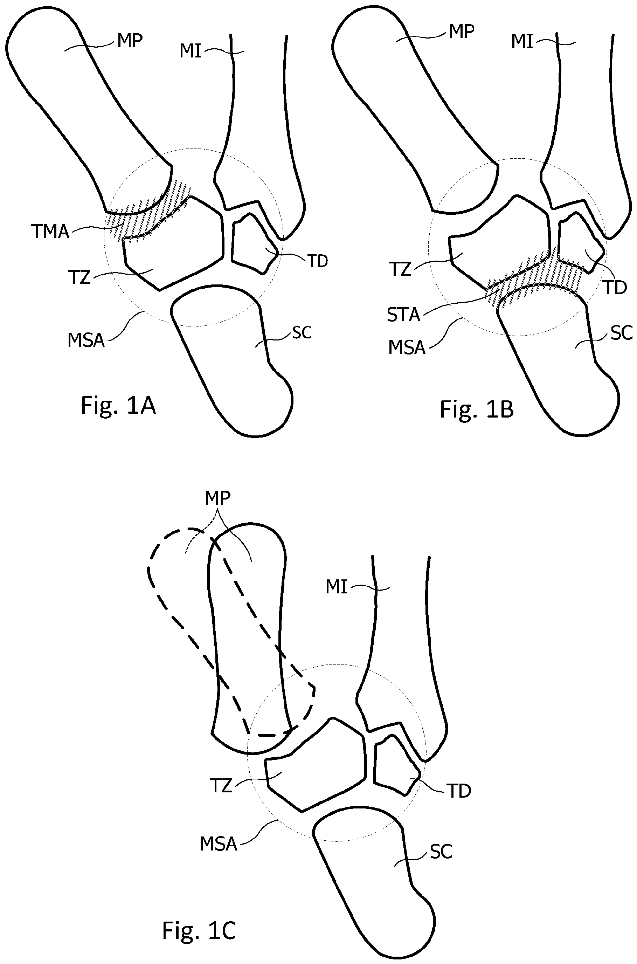

Les figures 1A, 1B, 1C représentent les articulations carpo-métacarpiennes du pouce et de l'index. Les articulations carpo-métacarpiennes du pouce MSA comprennent une articulation entre le métacarpe MP et le trapèze TZ, et une articulation entre d'une part le scaphoïde SC, et d'autre part, le trapèze TZ et le trapézoïde TD. Les articulations carpo-métacarpiennes de l'index comprennent une articulation entre le métacarpe MI de l'index et le trapézoïde TD et une articulation entre le trapézoïde TD et le scaphoïde SC. L'arthrose peut affecter l'une et/ou l'autre des articulations MSA, repérées par des hachures sur les figures 1A et 1B. Le pouce peut également être affecté d'une subluxation du métacarpe MP par rapport au trapèze TZ, comme illustré sur la figure 1C.

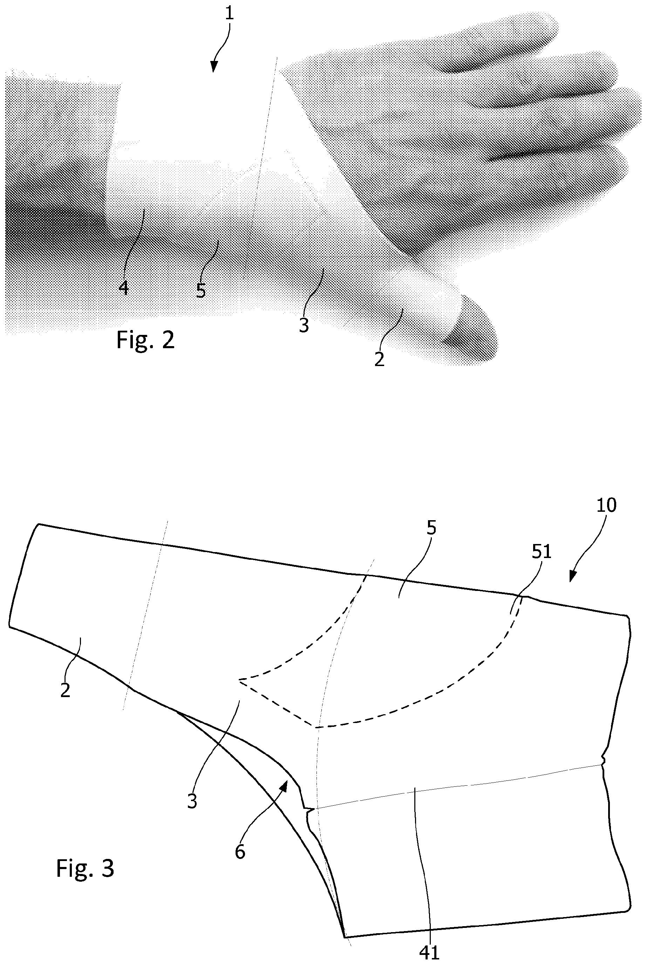

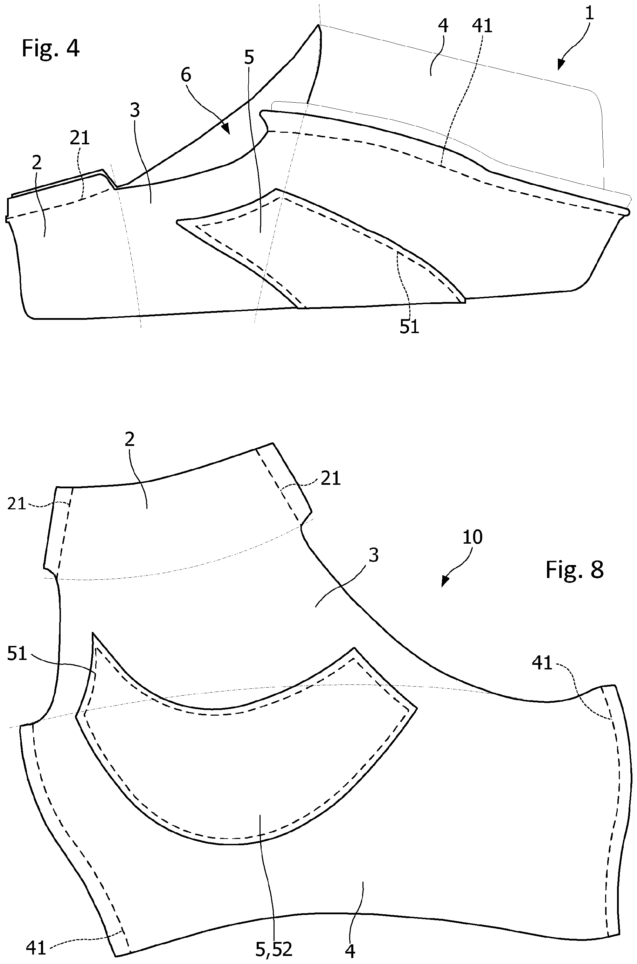

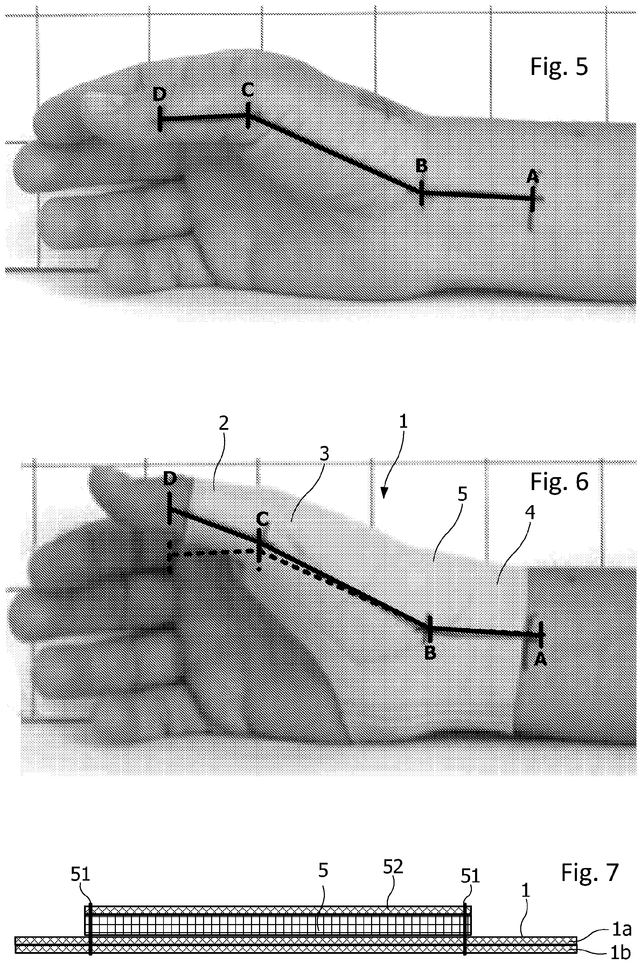

Les figures 2 à 4 et 6 représentent une orthèse de pouce, selon un mode de réalisation, visant à soulager les personnes souffrant d'une rhizarthrose ou d'une subluxation du métacarpe du pouce. Les figures 2 et 6 représentent l'orthèse placée sur une main. Les figures 3 et 4 représentent respectivement une partie de la face externe et de la face interne de l'orthèse. L'orthèse comprend une pièce principale 1 formant une partie distale 2, une partie proximale 4, et une partie intermédiaire 3 reliant les parties 2 et 4, délimitée sur les figures 2 à 4 par des lignes en trait mixte à points et tirets. La partie 2 en forme de manchon, est conformée pour couvrir et maintenir le pouce de la zone de pli entre le pouce et la paume de la main, à l'articulation inter-phalangienne du pouce. La partie 2 peut s'étendre par exemple jusqu'au voisinage du milieu de la phalange distale du pouce. La partie 4, également en forme de manchon, est conformée pour couvrir une partie de l'avant-bras, le poignet, les articulations carpo-métacarpiennes du pouce, et une partie proximale des métacarpes des doigts. La partie intermédiaire 3 présente une ouverture 6 s'étendant entre les parties 2 et 3, pour le passage de la paume de la main.

La pièce principale 1 est réalisée dans un matériau élastique et ajustée à la forme de la main et du pouce à maintenir, sans y exercer de force de contention trop intense, susceptible de provoquer des douleurs, en particulier dans la zone sensible des articulations carpo-métacarpiennes MSA. L'orthèse peut s'enlever en direction de l'extrémité des doigts et du pouce, sans effort important, notamment en raison de l'absence de zones d'étranglement.

Selon un mode de réalisation, le matériau formant la partie 1 présente une certaine rigidité qui se combine à un effet de poutre conféré par la forme tubulaire ou semi tubulaire à section droite courbe de l'orthèse. La rigidité résultante du matériau et de l'effet de poutre rend l'orthèse susceptible de supporter le poids du pouce sans fléchir.

Selon un mode de réalisation, le matériau formant la partie 1 présente un module de Young compris entre 0,5 MPa et 1 MPa, par exemple sensiblement égal à 0,7 MPa. L'effet de poutre obtenu peut être caractérisé par le moment quadratique. Ce moment est d'environ 3000 mm4 pour le manchon de pouce 2, et d'environ la moitié pour la partie intermédiaire 3.

La faculté de l'orthèse à supporter le poids du pouce est illustrée par les figures 5 et 6. Les figures 5 et 6 représentent la face interne d'une main posée sur son tranchant sur un plan horizontal, les muscles de main étant relâchés. Les axes de l'avant-bras, du métacarpe du pouce et de la phalange proximale du pouce sont repérés respectivement par des segments [A,B], [B,C] et [C,D]. Les articulations carpo-métacarpiennes MSA et métacarpo-phalangienne du pouce sont repérées par les points B et C. Dans la configuration de la main montrée sur la figure 5, il apparaît que les articulations B et C sont fléchies sous l'effet du poids du pouce. Le pouce se trouve ainsi sensiblement en dessous du niveau de l'index de la main sur la figure 5. Sur la figure 6, la main se trouve dans la même configuration que sur la figure 5, mais est équipée de l'orthèse. Il apparaît sur la figure 6 que le pouce se trouve à un niveau au dessus de l'index de la main. D'après la configuration des segments [B,C] et [C,D] de la figure 5 reproduite en trait pointillés sur la figure 6, l'angle ABC apparait légèrement plus petit sur la figure 6 que sur la figure 5. L'angle BCD apparait sensiblement plus grand (d'une vingtaine de degrés) sur la figure 6 que sur la figure 5.

Grâce à la rigidité de l'orthèse, les articulations B et C du pouce ne subissent pas de mouvements involontaires, par exemple durant des changements d'orientation de la main par rapport à la gravité, ou liés aux lois de la cinématique. Cette rigidité permet également de diminuer l'amplitude et la durée des mouvements involontaires comme les mouvements réflexes. En revanche, l'élasticité du matériau formant la partie 1 permet à l'orthèse de ne pas entraver les mouvements volontaires du pouce, et évite ainsi d'avoir à exercer des efforts supplémentaires importants sur l'articulation malade pendant de tels mouvements volontaires. L'utilisateur peut ainsi saisir un objet avec sa main en exerçant un effort supplémentaire minimum pour compenser la raideur de l'orthèse. Il est à noter sur les figures 2 et 6 que l'orthèse ne couvre pas la zone de pli entre le pouce et la paume de la main et donc n'entrave pas la fonction de pince de la main. A noter également que l'effet de soutien conféré par l'orthèse s'étend à l'articulation métacarpo-phalangienne C du pouce, cette articulation pouvant également souffrir d'arthrose.

Selon un mode de réalisation, le matériau dans lequel la pièce 1 est formée, est réalisé à l'aide de deux couches d'un tissu élastique, collées l'une sur l'autre, par une couche de colle d'environ 0,05 mm d'épaisseur. La couche de colle peut être uniformément répartie entre les deux couches de tissu, ou bien disposée par points uniformément répartis. Les deux couches de tissu peuvent être collées l'une contre l'autre en enduisant de colle l'une des deux couches, et en pressant les deux couches l'une contre l'autre, au moyen d'un ou deux rouleaux.

Le tissu formant les deux couches peut être un tissu à base de polyamide (environ 80% en poids) et d'élasthanne (environ 20% en poids), ayant un grammage de 155 g/m2. La colle utilisée peut être à base de polyuréthane. L'ensemble du matériau formé par les deux épaisseurs de tissu et de la couche de colle peut présenter un grammage de 355 g/m2.. Le tissu formant les deux couches peut présenter une épaisseur comprise entre 0,5 et 0,7 mm. Il en résulte que le matériau dans lequel la pièce 1 est formée peut présenter une épaisseur comprise entre 1 et 1,4 mm. Ce tissu peut présenter une élasticité comprise entre 85% à 115% dans le sens de la chaine et de 65% à 95% dans le sens de la trame.

Selon un mode de réalisation, l'orthèse comprend un élément amortissant 5 qui possède également une certaine raideur intrinsèque. L'élément amortissant 5 présente une forme adaptée et est disposé sur la partie principale 1 de manière à couvrir les articulations carpo-métacarpiennes du pouce. Sur les figures 2 à 4, l'élément amortissant présente sensiblement la forme d'un croissant dont les pointes sont tronquées (figure 8).

Selon un mode de réalisation, l'élément amortissant comprend une plaquette 5 en un matériau viscoélastique, tel qu'un gel polymère réticulé, par exemple un gel de silicone réticulé, non adhésif, qui est fixé sur la face intérieure de la partie 1.

La figure 7 représente une partie de l'orthèse sur laquelle est fixée la plaquette 5. Comme représenté sur la figure 7, la plaquette 5 peut être collée sur une pièce de tissu 52 qui est fixée sur la face interne de la partie principale 1 de l'orthèse par des coutures 51, la plaquette 5 étant prise en sandwich entre la pièce principale 1 et la pièce de tissu 52. La figure 7 montre également les deux couches 1a, 1b formant la pièce principale 1.

Selon un mode de réalisation, la pièce principale 1 peut être réalisée en une seule pièce 10 dont certains bords sont assemblés pour former l'orthèse. La figure 8 représente la pièce 10 à plat, comprenant les parties 2, 3, 4. Les parties 2 et 4 présentent sensiblement la forme d'une bande légèrement courbe, avec une ligne de couture 21, 41 à chaque extrémité. Les lignes 21 sont prévues pour être fixées l'une à l'autre pour former le manchon de pouce 2. De même, les lignes 41 sont fixées l'une à l'autre pour former le manchon de paume de main 4. La partie 3 présente une forme trapézoïdale sans ligne de couture. La figure 8 représente également la plaquette 5 dont la majeure partie est fixée sur la partie 4, et les parties restantes sur la partie 3, avant la réalisation des coutures 21, 41.

La pièce 10 peut être utilisée indifféremment pour réaliser des orthèses de main droite et de main gauche. La main à laquelle est destinée l'orthèse est déterminée par la face sur laquelle est fixée la plaquette 5. Ainsi, la pièce 10 représentée sur la figure 8 est destinée à réaliser une orthèse de main gauche. Les coutures 21 et 41 sont réalisées en pliant la pièce 10 vers la face externe de l'orthèse de manière à superposer les lignes 21 pour former le manchon 2, et à superposer les lignes 41 pour former le manchon 4.

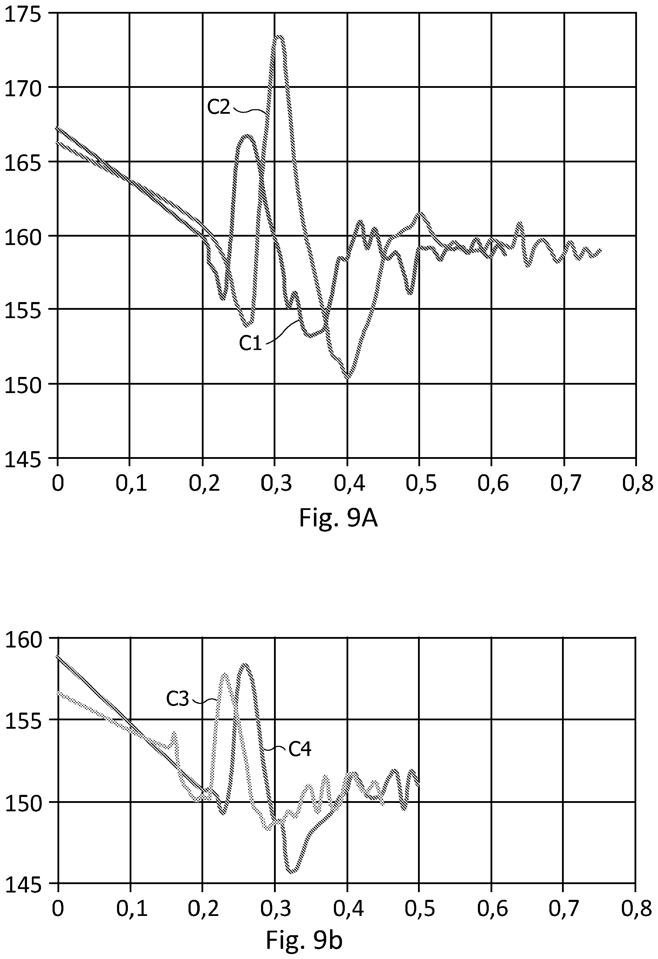

Les figures 9A et 9B représentent des courbes C1, C2, C3, C4 illustrant les performances de l'orthèse. Les courbes C1 à C4 montrent les variations en fonction du temps de l'angle ABC formé par les articulations carpo-métacarpiennes du pouce lors d'une chute sur un plan horizontal de la main, muscles relâchés. Les courbes C1 et C3 correspondent à une chute de 5 cm, et les courbes C2 et C4, une chute de 10 cm. Les courbes C1 et C2 sur la figure 9A illustrent le cas où la main ne porte pas d'orthèse et les courbes C3 et C4 sur la figure 9B illustrent le cas où la main porte l'orthèse telle que représentée sur les figures 2 à 4 et 6. D'après la courbe C1, l'angle ABC atteint un pic à 167° environ à la suite du choc à l'instant 0,22 s, puis oscille entre 154° et 161° d'une manière amortie. D'après la courbe C2, l'angle ABC atteint un pic à 173° environ à la suite du choc à l'instant 0,25 s, puis oscille entre 150° et 162° d'une manière amortie. D'après la courbe C3, le pic atteint par l'angle ABC est limité à 157° environ (au lieu de 163°) à la suite du choc à l'instant 0,21 s, puis oscille entre 148° et 152° d'une manière amortie. D'après la courbe C4, le pic atteint par l'angle ABC est limité à 158° environ (au lieu de 173°) à la suite du choc à l'instant 0,22 s, puis oscille entre 146° et 152° d'une manière amortie. Les courbes C1 à C4 font apparaître que les mouvements du pouce autour de l'articulation MSA, résultant du choc, présentent des amplitudes atténuées grâce à l'orthèse. Il peut également être observé que les oscillations résultant du choc durent moins longtemps avec l'orthèse.

Il est à noter que ces effets de maintien et d'amortissement sont obtenus sans que l'orthèse ne doive exercer des forces de contention en particulier à la jonction entre le poignet et la main. Il n'est donc pas nécessaire que l'orthèse soit ajustée exactement à la main de chaque personne à soulager. L'orthèse peut donc être fabriquée en quelques tailles standard. Ainsi, trois à cinq tailles standard peuvent être envisagées, sachant que 80% des cas peuvent être pris en compte avec seulement trois tailles standard.

Il apparaîtra clairement à l'homme de l'art que la présente invention est susceptible de diverses variantes de réalisation et diverses applications. En particulier, l'invention n'est pas limitée à une orthèse comprenant une plaquette d'amortissement. En effet, l'orthèse sans la plaquette assure parfaitement la fonction de maintien des articulations carpo-métacarpiennes du pouce de l'articulation. En outre, il peut être observé que si les oscillations observées sur la figure 9B présentent des amplitudes moins élevées que sur la figure 9A, ces oscillations durent également moins longtemps.

Il n'est pas non plus nécessaire de former la partie principale 1 à l'aide de deux couches de tissu élastique collées ensemble. D'autres matériaux peuvent être aisément trouvés pour donner une rigidité suffisante à la partie principale 1 de l'orthèse.

Il n'est pas non plus nécessaire que le manchon du pouce couvre l'articulation distale du pouce. En effet, le maintien des articulations carpo-métacarpiennes est parfaitement assuré lorsque l'orthèse ne couvre le pouce que jusqu'à la moitié de la phalange proximale. Par ailleurs, comme l'orthèse n'est réalisée qu'en quelques tailles standard et non sur mesure, la partie du pouce couverte par l'orthèse dépend des morphologies.

## Revendications

1. Orthèse de main pour le maintien du pouce, l'orthèse comprenant une pièce principale (1) réalisée en un matériau élastique et comportant :
une première partie (2) en forme de manchon ajustée à une partie du pouce s'étendant jusqu'à la zone de pli entre le pouce et la paume de la main,
une seconde partie (4) en forme de manchon ajustée au poignet, et
une troisième partie (3) reliant les première et seconde parties, conformée pour envelopper une zone de la main s'étendant le long du métacarpien du pouce entre le dos et la paume de la main, et comportant une ouverture (6) pour le passage de la paume de la main,
**caractérisée en ce que** la seconde partie (4) est assemblée par une ligne de couture pour prendre la forme d'un manchon, et la pièce principale (1) est conformée de manière à ne pas couvrir la zone de pli entre le pouce et la paume de la main et est réalisée en un matériau présentant dans une direction correspondant à un axe longitudinal de la première partie un module de Young compris entre 0,5 Mpa et 1 Mpa.

2. Orthèse selon la revendication 1, dans laquelle la première partie (2) est conformée pour couvrir l'articulation entre les deux phalanges du pouce.

3. Orthèse selon l'une des revendications 1 et 2, dans laquelle la pièce principale (1) comprend deux couches (1a, 1b) de tissu élastique assemblées par collage.

4. Orthèse selon la revendication 3, dans laquelle chacune des deux couches (1a, 1b) est réalisée dans un tissu comprenant entre 75 et 85% en poids de polyamide et entre 15 et 25% en poids d'élasthanne.

5. Orthèse selon l'une des revendications 3 et 4, dans laquelle chacune des deux couches (1a, 1b) présente un grammage de 155 g/m2, et/ou une épaisseur comprise entre 0,5 et 0,7 mm.

6. Orthèse selon l'une des revendications 3 à 5, dans laquelle chacune des deux couches (1a, 1b) présente une élasticité de 85% à 115% dans le sens de la chaine et de 65% à 95% dans le sens de la trame.

7. Orthèse selon l'une des revendications 1 à 6, comprenant une plaquette (5) en un matériau apte à assurer une fonction d'amortissement, la plaquette étant fixée à l'intérieur des seconde (4) et troisième (3) parties en une zone couvrant l'articulation entre le métacarpien du pouce et le poignet.

8. Orthèse selon la revendication 7, dans laquelle la plaquette (5) est réalisée en un gel polymère réticulé.

9. Orthèse selon l'une des revendications 7 et 8, dans laquelle la plaquette (5) présente une épaisseur comprise entre 0,4 et 1 mm.

10. Orthèse selon l'une des revendications 7 à 9, dans laquelle la plaquette (5) est collée sur une pièce de recouvrement (52) en tissu élastique qui est assemblée avec la pièce principale (1) par une couture (51), de sorte que la plaquette soit prise en sandwich entre la pièce principale et la pièce de recouvrement.

11. Orthèse selon la revendication 10, dans laquelle la plaquette (5) est cousue avec la pièce de recouvrement (52) sur la pièce principale (1).

12. Orthèse selon l'une des revendications 1 à 11, dans laquelle la pièce principale est ajustée à la main et au pouce pour maintenir les articulations du pouce dans une configuration de la main où tous les muscles de la main sont relâchés et en l'absence de forces extérieures s'exerçant sur les articulations du pouce.

13. Orthèse selon l'une des revendications 1 à 12, dans lequel la pièce principale (1) est réalisée en un matériau présentant une épaisseur comprise entre 1 mm et 1,4 mm.

## Patentansprüche

1. Orthese für eine Hand zum Halten des Daumens, wobei die Orthese ein Hauptteil (1) umfasst, das aus einem elastischen Material hergestellt ist und umfasst:
einen ersten Teil (2) in Form einer Hülse, die an einen Teil des Daumens angepasst ist, und sich bis zu einem Bereich der Falte zwischen dem Daumen und der Handfläche der Hand erstreckt,
einen zweiten Abschnitt (4) in Form einer Hülse, der an das Handgelenk angepasst ist, und
einen dritten Abschnitt (3), der den ersten und zweiten Teil verbindet und geformt ist, einen Bereich der Hand einzuwickeln, der sich entlang des Mittelhandknochens des Daumens zwischen dem Rücken und der Handfläche der Hand erstreckt und eine Öffnung (6) für den Durchgang der Handfläche der Hand aufweist,
**dadurch gekennzeichnet, dass** der zweite Teil (4) durch eine Nahtlinie verbunden ist, um die Form einer Hülse anzunehmen, und dass der Hauptteil (1) so geformt ist, um nicht den Bereich der Falte zwischen dem Daumen und der Handfläche der Hand zu bedecken und aus einem elastischen Material hergestellt ist, das in einer Richtung, die einer Längsachse des ersten Teils entspricht, ein Young-Modul zwischen 0,5 Mpa und 1 Mpa aufweist.

2. Orthese Anspruch 1, wobei der erste Teil (2) geformt ist, um das Gelenk zwischen den zwei Fingergliedern des Daumens zu bedecken.

3. Orthese nach einem der Ansprüche 1 und 2, wobei der Hauptteil (1) zwei Schichten (1a, 1b) aus einem elastischem Gewebe umfasst, die durch Kleben verbunden sind.

4. Orthese nach Anspruch 3, wobei jede der beiden Schichten (1a, 1b) aus einem Gewebe hergestellt ist, das zwischen 75 und 85 Gew.-% Polyamid und zwischen 15 und 25 Gew.-% Elastan umfasst.

5. Orthese nach einem der Ansprüche 3 und 4, wobei jede der beiden Schichten (1a, 1b) ein Flächengewicht von 155 g/m² und / oder eine Dicke aufweist, die zwischen 0,5 und 0,7 mm liegt.

6. Orthese nach einem der Ansprüche 3 bis 5, wobei jede der beiden Schichten (1a, 1b) eine Elastizität von 85 % bis 115 % in der Kettenrichtung und 65 % bis 95 % in der Richtung des Rasters aufweist.

7. Orthese nach einem der Ansprüche 1 bis 6, die eine Platte (5) aus einem Material umfasst, das geeignet ist, eine Dämpfungsfunktion sicherzustellen, wobei die Platte im Inneren des zweiten (4) und dritten (3) Teils in einem Bereich befestigt ist, der das Gelenk zwischen dem Mittelhandknochen des Daumens und dem Handgelenk abdeckt.

8. Orthese nach Anspruch 7, wobei die Platte (5) aus einem vernetzten Polymergel hergestellt ist.

9. Orthese nach einem der Ansprüche 7 und 8, wobei die Platte (5) eine Dicke zwischen 0,4 und 1 mm aufweist.

10. Orthese nach einem der Ansprüche 7 bis 9, wobei die Platte (5) auf einem Abdeckteil (52) aus elastischem Gewebe aufgeklebt ist, das mit dem Hauptteil (1) durch eine Naht (51) verbunden ist, so dass die Platte zwischen dem Hauptteil und dem Abdeckteil angeordnet ist.

11. Orthese nach Anspruch 10, wobei die Platte (5) mit dem Abdeckteil (52) auf dem Hauptteil (1) vernäht ist.

12. Orthese nach einem der Ansprüche 1 bis 11, wobei der Hauptteil an die Hand und den Daumen angepasst ist, um die Gelenke des Daumens in einer Konfiguration der Hand zu halten, in der alle Handmuskeln entspannt sind, und bei der Abwesenheit von äußeren Kräften, die auf die Gelenke des Daumens einwirken.

13. Orthese nach einem der Ansprüche 1 bis 12, wobei der Hauptteil (1) aus einem Material hergestellt ist, das eine Dicke zwischen 1 mm und 1,4 mm aufweist.

## Claims

1. A hand orthosis for supporting the thumb, the orthosis comprising a main part (1) made from an elastic material and comprising:
a first portion (2) in the form of a sleeve fitted to a portion of the thumb extending to the fold area between the thumb and the palm of the hand,
a second portion (4) in the form of a sleeve fitted to the wrist, and
a third portion (3) linking the first and second portions, shaped to envelop an area of the hand extending along the thumb metacarpal between the back and the palm of the hand, and comprising an opening (6) to allow the palm of the hand to pass therethrough,
**characterized in that** the second portion (4) is assembled by a seam line to take the form of a sleeve, and the main part (1) is shaped so as not to cover the fold area between the thumb and the palm of the hand and is made from a material having a Young's modulus of between 0.5Mpa and 1 Mpa in a direction corresponding to a longitudinal axis of the first portion.

2. The orthosis according to claim 1, wherein the first portion (2) is shaped to cover the joint between the two phalanges of the thumb.

3. The orthosis according to one of claims 1 and 2, wherein the main part (1) comprises two layers (1a, 1b) of elastic fabric assembled by adhesion.

4. The orthosis according to claim 3, wherein each of the two layers (1a, 1b) is made from a fabric comprising between 75 and 85% by weight of polyamide and between 15 and 25% by weight of elastane.

5. The orthosis according to one of claims 3 and 4, wherein each of the two layers (1a, 1b) has a weight per unit area of 155g/m2, and/or a thickness of between 0.5 and 0.7mm.

6. The orthosis according to one of claims 3 to 5, wherein each of the two layers (1a, 1b) has an elasticity of 85% to 115% in the direction of the warp and of 65% to 95% in the direction of the weft.

7. The orthosis according to one of claims 1 to 6, comprising a pad (5) made from a material capable of performing a cushioning function, the pad being set inside the second (4) and third (3) portions in an area covering the joint between the metacarpal of the thumb and the wrist.

8. The orthosis according to claim 7, wherein the pad (5) is made from a cross-linked polymer gel.

9. The orthosis according to one of claims 7 and 8, wherein the pad (5) has a thickness of between 0.4 and 1 mm.

10. The orthosis according to one of claims 7 to 9, wherein the pad (5) is adhered onto an overlapping part (52) made from an elastic fabric that is assembled with the main part (1) by a seam (51), such that the pad is sandwiched between the main part and the overlapping part.

11. The orthosis according to claim 10, wherein the pad (5) is sewn with the overlapping part (52) onto the main part (1).

12. The orthosis according to one of claims 1 to 11, wherein the main part is fitted to the hand and to the thumb to support the joints of the thumb in a hand configuration in which all the hand muscles are relaxed and in which no external forces are exerted on the joints of the thumb.

13. The orthosis according to one of claims 1 to 12, wherein the main part (1) is made from a material having a thickness of between 1 mm and 1.4mm.
